# EUROPÄISCHE PATENTSCHRIFT

(11) **EP 1 028 938 B1**
(45) Veröffentlichungstag und Bekanntmachung des Hinweises auf die Patenterteilung: **31.07.2002**
(21) Anmeldenummer: 98962225.3
(22) Anmeldetag: 29.10.1998
(51) Int. Cl.: C07C 227/08, C07C 229/30

(54) **HERSTELLUNG VON AMINOHALOGENCROTONATEN**
PRODUCTION OF AMINOHALOGENCROTONATES
PRODUCTION DE CROTONATES AMINOHALOGENES

(30) Priorität: 07.11.1997 DE 19749172; 07.11.1997 DE 19749171
(43) Veröffentlichungstag der Anmeldung: 23.08.2000
(73) Patentinhaber: Solvay Fluor und Derivate GmbH, 30173 Hannover (DE)
(72) Erfinder: BRAUN, Max, D-30900 Wedemark (DE); JANSSENS, Francine, B-1800 Vilvoorde (BE); RUDOLPH, Werner, D-30559 Hannover (DE); EICHHOLZ, Kerstin, D-30855 Langenhagen (DE)
(74) Vertreter: Gosmann, Martin, Dr.
(86) Internationale Anmeldenummer: DE9803263
(87) Internationale Veröffentlichungsnummer: WO9924390

(56) Entgegenhaltungen:
- DATABASE WPI Section Ch, Week 9506 Derwent Publications Ltd., London, GB; Class E16, AN 95-041245 XP002097791 & JP 06 321877 A (NISSAN CHEM IND LTD) , 22. November 1994 in der Anmeldung erwähnt
- HOUBEN-WEYL: "Methoden der organischen Chemie Band XI/1" 1957 , G. THIEME VERALG , STUTTGART XP002097790 siehe: Seiten 173,175 siehe Seite 170-175
- PASHKEVICH ET AL.: "Reactions of fluoroalkyl-beta-ketoesters with ammonia" BULL. ACAD. SCI. USSR DIV. CHEM.SCI. (ENGL.TRANSL.), Bd. 35, 1986, Seiten 1438-1442, XP002097881

## Beschreibung

Die Erfindung bezieht sich auf ein Verfahren zur Herstellung von am C-4-Atom durch Halogen substituierten Niedrigalkyl-3-amino-crotonaten, die an der Aminogruppe durch 1 oder 2 Wasserstoffatome und/oder 1 oder 2 C1-C3-Alkylgruppen oder 1 oder 2 Arylgruppen substituiert sind.

Am C-4-Atom durch Halogen substituierte Niedrigalkyl-3-amino-crotonate, insbesondere Niedrigalkyl-3-amino-4,4,4-trifluorocrotonate dienen als Baustein in der chemischen Synthese. Sie können beispielsweise für die Herstellung herbizider Uracil-Derivate eingesetzt werden, wie dies im US-Patent 5,399,543 beschrieben wird.

Ein Herstellungsverfahren für solche Verbindungen wird von W. F. Goure in J. Heterocyclic Chem. 30 (1993), Seiten 71 bis 80, insbesondere Seite 75 angegeben. Wasserfreies NH₃ wird während einer Zeitdauer von 1,5 Stunden bei einer Temperatur von 75 bis 80 °C durch 4,4,4-Trifluoracetessigsäure-Methylester durchgeleitet. Anschließend wurde die Reaktionsmischung noch 3 Stunden bei einer Temperatur von 100 °C belassen, dann auf Umgebungstemperatur abgekühlt, in Diethylether aufgenommen, getrocknet, konzentriert und einer Kugelrohrdestillation unterworfen. Die Ausbeute betrug 59 % der Theorie.

Die Thermolyse der Ammoniumsalze in Anwesenheit von Essigsäure und Ethanol wird in der japanischen Patentanmeldung JP 6/321877 beschrieben. Die europäische Patentanmeldung EP-A-0 808 826 offenbart die Herstellung von Trihalogencrotonaten unter Verwendung von Ammoniumsalzen als Aminquelle. Das Ammoniumsalz des Esters wird nicht gebildet. Die Bildung von Aminocrotonsäureestern aus NH3 und Acetessigsäureestern bzw. Trifluoracetessigsäureestern mit und ohne Lösungsmittel wird in Houben-Weyl, Methoden der organischen Chemie Band XI/I (1957), Seiten 170 - 175, und von Pashkevich et al. in Bull. Acad. Sci. USSR Div. Chem. Sci 35 (1986), Seiten 1438 - 1442 offnbart.

Aufgabe der vorliegenden Erfindung ist es, ein Verfahren zur Herstellung von am C-4-Atom durch Halogen substituierten Crotonaten ausgewählt aus der Gruppe umfassend Niedrigalkylester von 3-Amino-4,4,4-trifluorocrotonaten, Niedrigalkyl-3-amino-4,4-difluor-4-chlor-crotonaten und Niedrigalkyl-3-amino-4,4-difluorcrotonaten anzugeben, mit welchem diese Produkte in hoher Ausbeute mit hoher Reinheit erhalten werden. Diese Aufgabe wird durch das Verfahren der vorliegenden Erfindung gelöst.

Das erfindungsgemäße Verfahren zur Herstellung von am C-4-Atom durch Halogen substituierten Crotonaten ausgewählt . aus der Gruppe umfassend Niedrigalkyl-3-amino-4,4,4-trifluorocrotonate, Niedrigalkyl-3-amino-4,4-difluor-4-chlorcrotonate und Niedrigalkyl-3-amino-4,4-difluorcrotonate, wobei die Aminogruppe gegebenenfalls durch 1 oder 2 C1-C3-Alkylgruppen oder Arylgruppen substituiert sein kann und Niedrigalkyl Methyl, Ethyl, n-Propyl und i-Propyl bedeutet, sieht vor, daß man unter thermolytischen Bedingungen ohne Zusatz einer Säure die simultane Bildung von Wasser und am C-4-Atom durch Halogen substituierten Niedrigalkyl-3-amino-crotonaten bewirkt, und wobei man gebildetes Wasser aus der Reaktionsmischung abtrennt, indem man vom entsprechenden Ammoniumsalz des am C-4-Atom durch Halogen substituierten Acetessigsäure-Niedrigalkylesters ausgeht und in Anwesenheit eines Schleppmittels für das gebildete Wasser thermolysiert oder indem man in Abwesenheit eines Lösungsmittels Inertgas durch das geschmolzen vorliegende Ammoniumsalz des am C-4-Atom durch Halogen substituierten Acetessigsäure-Niedrigalkylesters leitet und dadurch gebildetes Wasser aus der Reaktionsmischung abführt.

Somit kann das Verfahren gemäß mehrerer Alternativen durchgeführt werden. Im folgenden ist die 1. Alternative erläutert.

Diese Alternative des erfindungsgemäßen Verfahrens zur Herstellung von am C-4-Atom durch Halogen substituierten Crotonaten ausgewählt aus der Gruppe umfassend Niedrigalkyl-3-amino-4,4,4-trifluorocrotonate, Niedrigalkyl-3-amino-4,4-difluor-4-chlor-crotonate und Niedrigalkyl-3-amino-4,4-difluorcrotonate, wobei die Aminogruppe gegebenenfalls durch 1 oder 2 C1-C3-Alkylgruppe oder Arylgruppen substituiert sein kann, ist dadurch gekennzeichnet, daß man das entsprechende Ammoniumsalz des am C-4-Atom halogensubstituierten Acetessigsäure-Niedrigalkylesters in Anwesenheit eines Schleppmittels für das bei der Thermolyse gebildete Wasser thermolysiert.

Gemäß dieser Ausführungsform kann man Niedrigalkylester des 3-amino-4,4-difluor-crotonats aus dem Ammoniumsalz (wobei der Stickstoff, insbesondere vollständig durch Wasserstoff substituiert ist oder durch Wasserstoff und 1 oder durch 2 C1-C3-Alkylgruppen substituiert sein kann) des 4,4-Difluoracetessigsäureesters herstellen. Niedrigalkylester des 3-Amino-4,4-difluor-4-chlor-crotonates stellt man entsprechend aus dem Salz des 4,4-Difluor-4-chlor-acetessigesters her. Entsprechend geht man zur Herstellung von Niedrigalkylestern des 3-Amino-4,4,4-trifluorocrotonats vor.

Die benötigten Ester sind Handelsprodukte oder können durch Umsetzung von Keten mit Trifluor-, Difluorchlor- bzw. Difluoracetylchlorid und anschließender Veresterung hergestellt werden, wie bei W. F. Goure, J. Heterocyclic Chem. 30 (1993), Seiten 71 bis 80, insbesondere Seite 72, beschrieben.

Das erfindungsgemäße Verfahren kann so durchgeführt werden, daß man vom Ammoniumsalz ausgeht. Beispielsweise kann man es in einer 1. Stufe vorab herstellen. In einer 2. Stufe wird es dann thermolysiert. Gemäß einer anderen Ausführungsform stellt man das Ammoniumsalz in situ her und thermolysiert es simultan. Diese Ausführungsform kann auch kontinuierlich durchgeführt werden.

Bevorzugt stellt man 4,4,4-Trifluorcrotonate her. Anhand dieser bevorzugten Ausführungsform wird die Erfindung weiter erläutert.

Generell gilt für die vorliegende Erfindung in allen Alternativen: ein Zusatz katalysierender Mittel, wie beispielsweise Säure, ist nicht notwendig und erfolgt nicht. Das verwendete Amin bzw. NH₃ wird als Base und nicht als Salz, z.B. von Carbonsäuren, eingesetzt. Der Begriff "Niedrigalkyl" steht für Methyl, Ethyl, n-Propyl und i-Propyl. "Aryl" bedeutet vorzugsweise Phenyl.

Bevorzugt stellt man Crotonate her, welche an der Aminogruppe zwei Wasserstoffatome tragen. Ganz besonders bevorzugt stellt man Methyl-3-amino-4,4,4-trifluorocrotonat oder Ethyl-3-amino-4,4,4-trifluorocrotonat her.
Man verwendet Schleppmittel, die weder mit den Ausgangsverbindungen noch mit den Produkten unerwünschte Reaktionen eingehen. Geeignet sind beispielsweise aliphatische, aromatische Kohlenwasserstoff oder halogenierte aliphatische Kohlenwasserstoffe. Beispielsweise kann man Benzol oder Toluol als Schleppmittel einsetzen. Besonders bevorzugt sind jene Schleppmittel, die spezifisch schwerer als die sich ausbildende Wasserphase sind und deshalb die untere Phase darstellen. Besonders gut geeignet sind halogenierte Kohlenwasserstoffe wie Trichlorethylen oder Tetrachlorkohlenstoff. Spezifisch schwerere Wasserschleppmittel extrahieren Ammoniumsalz, welches in der leichteren Wasserphase im Wasserabscheider vorhanden ist, und vermindern auf diese Weise den Ausbeuteverlust, insbesondere bei sublimierenden Produkten.

Man arbeitet unter Bedingungen, in welchen das Wasserschleppmittel siedet. Der Einfachheit halber arbeitet man bei Umgebungsdruck; gewünschtenfalls kann man natürlich auch bei erhöhtem oder vermindertem Druck arbeiten.

Bevorzugt liegt die Temperatur in der Reaktionsmischung im Bereich von 80 bis 120 °C. Natürlich hängt die Temperatur, die man anwendet, auch ab vom Siedepunkt des Schleppmittels.

Nach Beendigung der Wasserabscheidung wird das Schleppmittel, beispielsweise durch Destillation, abgetrennt und das verbleibende rohe Crotonat durch Feindestillation im Vakuum (beispielsweise Wasserstrahlvakuum) gereinigt. Ethyl-3-amino-4,4,4-trifluorcrotonat beispielsweise destilliert bei 55 bis 57 °C.

Gemäß der zweiten Alternative, die im folgenden beschrieben wird, führt man das Reaktionswasser durch Inertgas ab. Diese Ausführungsform zur Herstellung von am C-4-Atom durch Halogen substituierten Niedrigalkyl-3-amino-crotonaten ausgewählt aus der Gruppe umfassend Niedrigalkyl-3-amino-4,4,4-trifluorocrotonate, Niedrigalkyl-3-amino-4,4-difluorcrotonate und Niedrigalkyl-3-amino-4,4-difluor-4-chlorcrotonate, sieht vor, daß man in Abwesenheit eines Lösungsmittels vom geschmolzen vorliegenden Ammoniumsalz des am C-4-Atom durch Halogen substituierten Acetessigsäure-Niedrigalkylesters ausgeht, unter thermolytischen Bedingungen die simultane Bildung von Wasser und am C-4-Atom durch Halogen substituierten Niedrigalkyl-3-amino-crotonaten bewirkt und durch Leiten von Inertgas durch das geschmolzene Salz, das bei der Thermolyse gebildete Wasser aus der Reaktionsmischung abführt. Das Stickstoffatom kann durch 1 oder 2 C1-C3-Alkylgruppen oder Arylgruppen substituiert sein.

Entsprechend kann man Niedrigalkylester des 3-Amino-4,4-difluor-crotonats aus dem Ammoniumsalz des 4,4-Difluor-acetessigsäureesters herstellen. Niedrigalkylester des 3-Amino-4,4-difluor-4-chlor-crotonates stellt man entsprechend aus dem Salz des 4,4-Difluor-4-chlor-acetessigesters her. Entsprechend geht man zur Herstellung von Niedrigalkylestern des 3-Amino-4,4,4-trifluorcrotonats vor.

Die benötigten Ester sind Handelsprodukte oder können durch Umsetzung von Keten mit Trifluor-, Difluorchlor- bzw. Difluoracetylchlorid und anschließender Veresterung hergestellt werden, wie bei W. F. Goure, J. Heterocyclic Chem. 30 (1993), Seiten 71 bis 80, insbesondere Seite 72 beschrieben.

Der Begriff "Niedrigalkyl" steht für die Methyl-, Ethyl-, n-Propyl- und i-Propyl-Gruppe. Bevorzugt steht Niedrigalkyl für Methyl oder Ethyl. "Aryl" bedeutet vorzugsweise Phenyl.

Bevorzugt thermolysiert man bei einer Temperatur im Bereich vom Schmelzpunkt des Salzes bzw. der Reaktionsmischung bis hin zu einer Temperatur von maximal 110 °C, insbesondere maximal 105 °C. Man kann auch noch bei höheren Temperaturen arbeiten, beispielsweise bis hin zu 120 °C oder höher, dabei kann es jedoch in geringem Umfang zur Bildung von Zersetzungsprodukten kommen. Sehr gute Ergebnisse werden erzielt, wenn die Temperatur der Reaktionsmischung im Bereich von 80 bis 95 °C liegt. Als Inertgas kann man Gase verwenden, die mit der Ausgangsverbindung und den gebildeten Produkten nicht in unerwünschter Weise reagieren. Vorteilhaft wendet man als Inertgas Stickstoff, Argon, He oder CO₂ oder deren Gemische an.

Bevorzugt stellt man 4,4,4-Trifluorcrotonate her. Anhand dieser bevorzugten Ausführungsform wird die Erfindung weiter erläutert.

Das erfindungsgemäße Verfahren kann man auch in dieser Variante gemäß einer Ausführungsform so durchführen, daß man zunächst das Ammoniumsalz des 4,4,4-Trifluoracetessigsäure-Niedrigalkylesters durch Einleiten von im wesentlichen wasserfreiem Ammoniak in und/oder über den 4,4,4-Trifluoracetessigsäure-Niedrigalkylester herstellt. In einer zweiten Stufe kann dann die Thermolyse unter Schmelzen des gebildeten Salzes (ohne daß eine weitere Reinigungsoperation notwendig wäre) erfolgen. Umsatz und Ausbeute sind quantitativ. Das gebildete Crotonat kann aufgrund seiner Reinheit ohne weitere Reinigungsoperation als Synthesebaustein, beispielsweise bei der Herstellung von Herbiziden, eingesetzt werden.

Gemäß einer anderen Ausführungsform der Erfindung wird auch in dieser Variante die Bildung des Ammoniumsalzes des 4,4,4-trifluoracetessigsäure-Niedrigalkylesters und seine Thermolyse simultan durchgeführt. Zweckmäßig geht man so vor, daß man den Trifluoracetessigsäure-Niedrigalkylester vorlegt und erhitzt und im wesentlichen wasserfreien Ammoniak in und/oder über den Ester leitet sowie Inertgas durch die Reaktionsmischung durchleitet. Beispielsweise kann man einen rohrförmigen Reaktor verwenden und Ammoniak am Reaktorboden einleiten. Den Ester kann man beispielsweise im oberen Bereich des Reaktors einbringen. Das Verfahren kann auch kontinuierlich durchgeführt werden.

Der Passus "In Abwesenheit eines Lösungsmittels" besagt, daß außer den Reaktanten kein Lösungsmittel wie Ether, Ester, aromatische oder Halogenkohlenwasserstoffe zugefügt werden. Lediglich der eingesetzte am C-4-Atom halogenierte Acetessigsäure-Niedrigalkylester kann im Überschuß vorliegen. In diesem Fall kann das sich bildende Ammoniumsalz geschmolzen und/oder gelöst im Ester vorliegen. Diese Ausführungsform wird vom erfindungsgemäßen Verfahren umfaßt. Der Begriff "geschmolzen" umfaßt also auch den Zustand der Lösung des Ammoniumsalzes im Ester-Edukt. Zur Vervollständigung der Reaktion kann man nach Beendigung der Ammoniakeinleitung (der Zeitpunkt der Beendigung der Ammoniakeinleitung kann durch beispielsweise NMR-Analyse überprüft werden) die thermolytischen Bedingungen noch eine Zeitlang weiter aufrechterhalten. Der Zeitpunkt, zu dem eine vollständige Umsetzung erfolgt ist, kann durch Untersuchung mittels NMR-Analyse ermittelt werden. Das recht rein anfallende Produkt kann gewünschtenfalls durch Destillation weiter gereinigt werden. Selbst wenn man eine solche Destillation durchführt, ist die Ausbeute immer noch bemerkenswert hoch.

Die Produkte fallen rein an. Eine destillative Reinigung kann vorgenommen werden. Extraktion des Rohproduktes mittels Lösungsmittel, wie im Stand der Technik beschrieben, ist bei der Aufarbeitung nicht nötig.

Das erfindungsgemäße Verfahren gestattet die Herstellung von Niedrigalkyl-3-amino-4, 4, 4-trifluorcrotonaten in einfacher Vorgehensweise, mit hoher Ausbeute und in hoher Reinheit. Dabei ist es erstaunlich, daß die Wasserabtrennung Vorteile bringt. Es handelt sich nämlich bei der dehydratisierenden Thermolyse um keine Gleichgewichtsreaktion. Andernfalls müßte bei der wäßrigen Aufarbeitung eine Rückreaktion beobachtet werden. Dies ist aber offensichtlich nicht der Fall.

Die folgenden Beispiele sollen die Erfindung weiter erläutern ohne sie in ihrem Umfang einzuschränken.

### Beispiele

### Versuchsvorschrift zur Herstellung von 3-Amino-4,4,4-trifluorethyl-crotonat aus 4,4,4-Trifluoracetessigsäureethylester mit Ammoniak unter Verwendung verschiedener Wasserschlepper (Schleppmitteln)

### Beispiele 1 und 2:

Durchführung mit spezifisch leichteren Wasserschleppern

### Beispiele 3 und 4:

Durchführung mit spezifisch schwereren Wasserschleppern

### Ansatz (gilt für Beispiele 1 bis 4):

92,05 g (0,50 mol) 4,4,4-Trifluoracetessigsäureethylester
14,40 g (0,85 mol) Ammoniak
125 ml Lösemittel

### Aufbau und Durchführung:

### Beispiel 1: Benzol als Wasserschlepper

In einem 250 ml Dreihalskolben mit Thermofühler, Wasserabscheider und Magnetrührer wurde der 4,4,4-Trifluoracetessigsäureethylester vorgelegt und mit Benzol bis zu einem ca. 80%igen Füllstand im Kolben aufgefüllt. Der angeschlossene Wasserabscheider (Volumen ca. 40 ml) für spezifisch leichtere Wasserschlepper, in dem die untere Phase die Wasserphase darstellen wird, wurde ebenfalls vollständig mit Benzol gefüllt, um die Anlaufzeit des Versuches zu verkürzen.

Dann wurde die Lösung auf Siedetemperatur Benzol erhitzt, bis ein gleichmäßiger Rückfluß entstand. Bei dieser Temperatur wurde über 30 min. der Ammoniak eingeleitet, die Reaktion ist exotherm. Die klare Lösung trübte sich im Fortgang der Reaktion ein (es entsteht zunächst das intermediare Ammoniumsalz NH₄' [CF₃COCHCO₂Et]⁻). Dieses Ammoniumsalz neigte während der Reaktion zur Sublimation (Niederschlag am Rückflußkühler des Wasserabscheiders). Nach Beendigung der Ammoniakeinleitung wurde noch 1 Stunde nachgekocht. Der Sumpf und die obere Phase des Wasserabscheiders bestanden nach dem Abkühlen nun aus Benzol und dem gewünschten Produkt. In der Wasserphase war noch teilweise Ammoniumsalz gelöst. Der Umsatz zum Crotonat betrug 91 %.

### Beispiel 2: Toluol als Wasserschlepper

Die Durchführung erfolgte wie bei Beispiel 1, nur bei 110 °C. Der Umsatz des 4,4,4-Trifluoracetessigsäureethylesters zum Crotonat war quantitativ.

### Beispiel 3: Trichlorethylen als Wasserschlepper

Die Durchführung erfolgte wie bei Beispiel 1, nur bei 93 °C, und es wurde ein Wasserabscheider für spezifisch schwerere Wasserschlepper benutzt, bei dem die Wasserphase als obere Phase abgenommen wurde. In die Wasserphase während der Reaktion durch Sublimation mitgeschlepptes und gelöstes Ammoniumsalz wurde hierbei durch permanente Extraktion durch den Wasserschlepper wieder in den Reaktionskolben zurückgeführt. Es wurde nach insgesamt 4 Stunden Kochen ein Umsatz von 99 % des 4,4,4-Trifluoressigsäureethylesters zum Crotonat erreicht.

### Beispiel 4: Tetrachlorkohlenstoff als Schleppmittel

Die Durchführung erfolgte wie bei 3, jedoch bei 85 °C. Der Umsatz betrug 86,35 %.

### Aufarbeitung des Produktes (gilt für alle Versuche):

Reines Crotonat wurde in allen Beispielen durch anschließende Vereinigung der Sümpfe mit den Schlepperphasen und Abrotieren des Wasserschleppers und Feindestillation im Wasserstrahivakuum erhalten, wobei das 3-Amino-4,4,4-Trifluorethylcrotonat bei 55 bis 57 °C überging. Die isolierten Ausbeuten lagen zwischen 80 und 92 % d. Th.

### Beispiele 5 bis 9: Herstellung unter Inertgaseinleitung

### I) Versuchsvorschrift zur einstufigen Darstellung von Ethyl-3-amino-4,4,4-trifluorocrotonat aus 4,4,4-Trifluoracetessigsäureethylester und Ammoniak

### Apparatur für die Beispiele 5 - 7:

Die Apparatur besteht aus einem 200 mm langen mit einem Doppelmantel (für Thermoölbeheizung) versehenen Glasrohr (Durchmesser 25 mm) mit oben aufgeschmolzenem 250 ml Dreihalskolben, auf dem ein auf 50 °C temperierter Rückflußkühler aufgesetzt ist. Der Innenraum des Glasrohres wird vollständig mit Edukt gefüllt. Ca. 50 mm unterhalb des aufgeschmolzenen Dreihalskolbens befindet sich eine in den Innenraum des Glasrohres reichende Fritte, über die während des Versuchsbetriebes Stickstoff zum Ausblasen des entstehenden Reaktionswassers eingeführt wird. Die Ammoniakeinspeisung erfolgt über eine Fritte installiert am Boden des Glasrohres.

### Beispiel 5:

### Ansatz:

226,8 g (1,23 mol) 4,4,4-Trifluoracetessigsäureethylester
22,1 g (1,30 mol) Ammoniak

### Aufbau u. Durchführung:

In das Reaktionsrohr wurden 226,8 g (1,23 mol) 4,4,4-Trifluoracetessigsäureethylester vorgelegt und mit einem Thermostaten auf ca. 100 °C temperiert (Innentemperatur). Nun wurde die N₂-Ausblasung mit ca. 100 l/h angestellt und 22,1 g (1,23 mol) Ammoniak über insgesamt 48 Minuten eingeleitet. Nach 5,0 g; 10,8 g; 15,4 g und 21,8 g Ammoniak wurde jeweils eine Flüssigprobe für die NMR-Analytik aus dem oberen Bereich der Apparatur genommen. Eine Reaktionsprobe, genommen bei 50 % Zugabe an NH₃ entsprechend der Ammoniakstöchiometrie, zeigt deutlich die Bildung des 3-Amino-4,4,4-trifluorethylcrotonats an. Die wasserhaltige Vorstufe des Crotonates NH₄⁺(CF₃COCHCO₂Et)⁻ war in dieser Probe zu ca. 10 % vorhanden. Nach Beendigung der Ammoniakeinleitung betrug der Ammoniumsalzanteil noch ca. 13 %. Aus diesem Grund wurde noch 30 min. weiter gekocht. Nach dieser Zeit war alles Ammoniumsalz zu 3-Amino-4, 4, 4-trifluorethylcrotonat umgesetzt. Anschließend wurde bei 15 bis 18 mbar fraktioniert destilliert, wobei nach einem Edukt-Vorlauf das reine Produkt bei 56 bis 57 °C als farblose Flüssigkeit erhalten wurde, die bei Raumtemperatur (Schmelzpunkt: 26 °C) erstarrte. Die isolierte Crotonat-Ausbeute betrug 84 % d. Th.

### Beispiel 6:

Durchführung wie Beispiel 5, jedoch bei 90 °C:

Bei einer Reaktionstemperatur von 90 °C waren nach der Beendigung des Ammoniakeinleitens noch 38 % und nach 30 min. Nachkochen immer noch 14 % Ammoniumsalz vorhanden. Nach 1,5 h Nachkochen war schließlich die Ammoniumsalz-Zwischenstufe vollständig zu Crotonat umgesetzt.

### Beispiel 7:

Durchführung wie Beispiel 5, jedoch bei 110 °C:
Bei einer Reaktionstemperatur von 110 °C war nach der Beendigung des Ammoniakeinleitens zwar das Ammoniumsalz vollständig zu Crotonat umgesetzt, jedoch wurde im Stickstoffstrom neben Ethanol auch Trifluoraceton nachgewiesen, die auf eine beginnende Zersetzung des Eduktes hinweisen.

### II) Versuchsvorschrift zur zweistufigen Darstellung von Ethyl-3-amino-4,4,4-trifluorocrotonat aus 4,4,4-Trifluoracetessigsäureethylester und Ammoniak über das Ammoniumsalz des 4,4,4-Trifluoracetessigsäure-ethylesters (NH₄⁺[CF₃COCHCO₂Et]⁻ )

### Beispiel 8:

### 1. Stufe: Herstellung von NH₄⁺[CF₃COCHCO₂Et]⁻:

| Ansatz: | | |
|---|---|---|
| 4,4,4-Trifluoracetessigsäureethylester (ETFAA) | 210,0 g | (1,14 mol) |
| Ammoniak | 15,3 g | (0,90 mol) |

### Aufbau und Durchführung:

In einem 250 ml Mehrhalskolben mit KPG-Rührer, Thermofühler und Trockeneiskühler wurde der ETFAA vorgelegt und unter starkem Rühren wurde sehr langsam Ammoniak eingeleitet. Sofort trübte sich die Lösung ein und Ammoniumsalzkristalle wurden sichtbar. Die Reaktion war exotherm, mit einem kalten Wasserbad wurde deshalb gegengekühlt und die Einleitgeschwindigkeit soweit zurückgenommen, so daß eine Temperatur von 40 °C nicht überschritten wurde. Die Suspension wurde immer breiiger und fester, deshalb wurde nicht bis zur Stöchiometrie eingeleitet.

Die so erhaltenen Kristalle wurden über einen Blaubandfilter abgesaugt, der im Kolben verbleibende Rest wurde mit ca. 2 x 10 ml Waschlösung (Hexan: Essigester 9:1) überführt. Der Filterkuchen wurde nochmals aufgerührt und mit 3 x 10 ml. o. g. Waschlösung gereinigt, um den überschüssigen als Solvens verwendeten ETFAA auszuwaschen. Das Erscheinungsbild des Ammoniumsalzes waren weiße, feine Kristalle, die erst nach dem Waschen eine pulvrige Konsistenz bekamen. Nach dieser Vorgehensweise wurden 170,3 g Ammoniumsalz mit 99 % Reinheit erhalten. Das entspricht einer isolierten Ausbeute von 94,09 % der Theorie. Die Analytik der Kristalle und die Reinheitsbestimmung erfolgte mittels NMR.

### NMR-Daten:

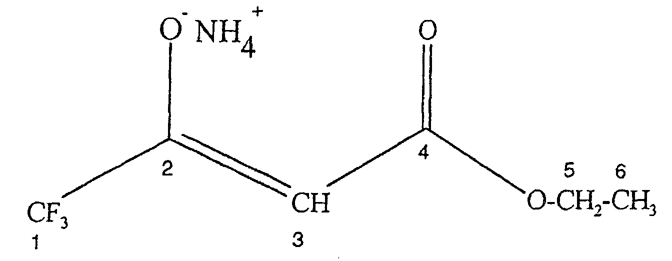

Die NMR Daten von der Substanz zeigen folgende chemische Verschiebungen:
¹³C- NMR ( 100 MHz, DMSO): δ (ppm) = 119,9 ( C-1); 166,1 (C-2); 77,9 (C-3); 167,8 (C-4); 56,6 (C-5); 14,7 (C-6). Ebenso, wie die ¹³C Daten, waren die 400 MHz ¹H im Einklang mit der Sollstruktur.

### Beispiel 9:

### 2. Stufe: Thermolyse des NH₄⁺[CF₃COCHCO₂Et]⁻ zum 3-Amino-4,4,4-trifluorethylcrotonat:

In einem 100 ml Dreihalskolben mit N₂-Einspeisung wurden 50 g Ammoniumsalz eingefüllt. Der Kolben wurde mit einem Rückflußkühler versehen und in ein auf 120 °C temperiertes Ölbad gegeben und der Stickstoffstrom durch die Schmelze zum Austreiben des Wassers angestellt. Nach 7 Minuten war das Salz vollständig geschmolzen (T= 84 °C bei Probenahme), aus der Schmelze wurde eine Startprobe für NMR-Analyse genommen. Zu diesem Zeitpunkt betrug der Umsatz zum Crotonat bereits 38 %. In 15 min. Abstand wurden weitere Proben zur Umsatzkontrolle genommen. Nach 60 min. betrug der Umsatz 98 %, nach 90 min. war kein Ammoniumsalz mehr nachweisbar. Die Ausbeute an 3-Amino-4,4,4-trifluorethylcrotonat war quantitativ. Das Crotonat kann aufgrund der selektiven Reaktion ohne weitere Reinigung für nachfolgende Synthesen eingesetzt werden.

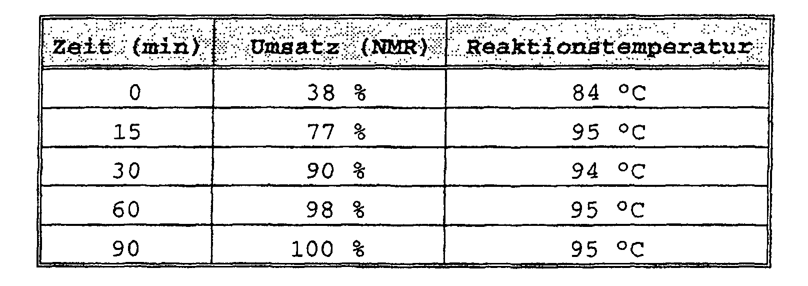

Die Tabelle zeigt, daß bereits nach 90 Minuten ein quantitativer Umsatz erreicht war, wobei selektiv das gewünschte Produkt entstand.

### NMR-Daten:

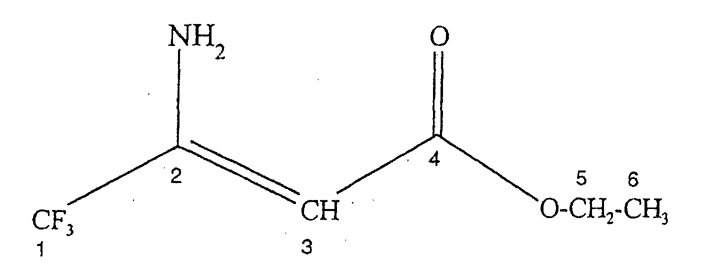

Die NMR Daten von der Substanz zeigen folgende chemische Verschiebungen:
¹³C- NMR ( 100 MHz, DMSO): δ (ppm) = 120,6 ( C-1); 147,6 (C-2); 82,6 (C-3); 168,2 (C-4); 59,1 (C-5); 14,2 (C-6).
Ebenso, wie die ¹³C Daten, waren die 400 MHz ¹H im Einklang mit der Sollstruktur.

### Beispiel 10:

Einstufige Synthese von Trifluorethyl- methylaminocrotonat aus 4,4,4 Trifluoracetessigsäureethylester und Methylamin

### Ansatz:

1,0 mol Trifluoracetessigsäureethylester (ETFAA ) 184,01 g
1,0 mol Methylamin 31,05 g

### Apparatur:

Die Apparatur besteht aus einem 200 mm langen mit einem Doppelmantel (für Thermoölbeheizung) versehenen Glasrohr (Durchmesser 25 mm) mit oben aufgeschmolzenem 250 ml Dreihalskolben auf dem ein auf 70 °C temperierter Rückflußkühler aufgesetzt ist. Der Innenraum des Glasrohres wird vollständig mit Edukt gefüllt. Ca. 50 mm unterhalb des aufgeschmolzenen Dreihalskolbens befindet sich eine in den Innenraum des Glasrohres reichende Fritte über die während des Versuchsbetriebes Stickstoff zum Ausblasen des entstehenden Reaktionswassers eingeführt wird. Die Amineinspeisung erfolgt über eine Fritte installiert am Boden des Glasrohres.

### Aufbau u. Durchführung:

In der Apparatur (die auch zur Herstellung des nichtsubstituierten Crotonats eingesetzt wurde) wurde der ETFAA vorgelegt und auf 115 °C temperiert, dann wurde über die untere Glasfritte eine Mischung aus 10 Teilen Stickstoff und einem Teil Methylamin (2 g/min) eingeleitet. Dieses Vermischung des Amins mit Inertgas vor Eintritt in den Reaktor ermöglicht neben dem Schleppeffekt des Inertgases eine unproblematische Reaktionsführung, da ohne Stickstoffbeimischung die Einleitestelle sofort wegen der Bildung des entsprechenden Ammonioumsalzes verstopft und sich Nebenprodukte durch eine lokale Überhitzung bilden können. Alle 20 Minuten wurde die Methylamin-Dosierung kurz gestoppt um das Ammoniumsalz vollständig zu thermolysieren. In dieser Zeit wirkte der Stickstoff nur als Schleppgas für das entstehende Wasser. Das Abgas der Apparatur wurde durch den auf 70 °C temperierten Kühler in eine nachgeschaltete Kühlfalle geleitet. Sublimierendes Ammoniumsalz wurde durch mitgeschleppten ETFAA wieder heruntergewaschen und der Reaktion so wieder zugeführt. Nach Beendigung des Einleitens wurde noch weitere 2 Stunden mit einem Stickstoffstrom von ca. 30 L/h, bei 115 °C, beaufschlagt. Nach dieser Zeit hatten sich die noch vorhandenen Ammoniumsalzkristalle zersetzt. Die NMR Probe zeigte nach dieser Zeit einen Umsatz von 98 % an. In der Kühlfalle nach der Apparatur wurden neben dem Wasser auch Spuren von Ethanol und TFK-Hydrat (entstanden durch Decarboxylierung von ETFAA zusammen mit Feuchtigkeit) nachgewiesen. 20 g von der nach dem Abkühlen öligen, bernsteinfarbenen Substanz wurden anschließend einer Kugelrohrdestillation unterzogen. Bei einem Vakuum von 15 mbar und einer Temperatur im Kugelrohrofen von 75 - 80 °C ging reines, N-methylsubstituiertes Crotonat über.

### NMR-Daten:

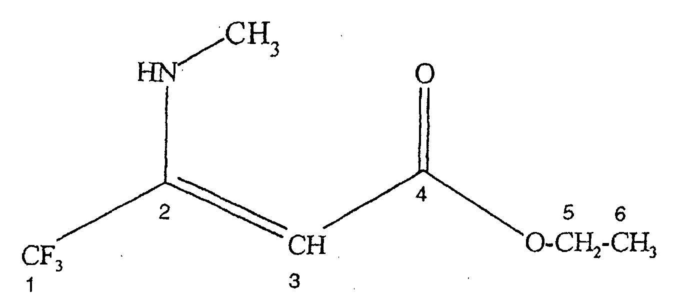

Die NMR Daten von der Substanz zeigen folgende chemische Verschiebungen:
¹³C- NMR ( 100 MHz, CDCl₃): δ (ppm) = 120/3 ( C-1); 149,2 (C-2); 84,2 (C-3); 169,9 (C-4); 59,5 (C-5); 14,1 (C-6); 30,3 (C-7). Ebenso, wie die ¹³C Daten, waren die 400 MHz ¹H im Einklang mit der Sollstruktur.

### Beispiel 11:

Einstufige Synthese von Trifluorethyl-phenylaminocrotonat aus 4,4,4 Trifluoracetessigsäureethylester und Anilin

### Ansatz:

1,0 mol Trifluoracetessigsäureethylester (ETFAA ) 184,01 g
1,0 mol Anilin ( frisch destilliert ) 93,1 g

### Aufbau u. Durchführung:

In einem 250 ml Mehrhalskolben, mit einem auf 70 °C temperierten Rückflußkühler und nachgeschalteter Kühlfalle, wird der ETFAA vorgelegt und auf 100 °C temperiert. Bei dieser Temperatur wird langsam und unter starkem Rühren Anilin zugetropft, gleichzeitig wird durch eine Glasfritte Stickstoff in die Lösung geblasen(ca.50 l/h).

Die Reaktion war leicht exotherm, und sofort nach Beginn des Zutropfens war die Ammoniumsalzbildung erkennbar. In der Wärme zersetzten sich die Kristalle nach kurzer Zeit zu Wasser und Crotonat. Durch den Schleppeffekt des Stickstoffes wurde das entstehende Wasser sofort aus dem Gleichgewicht entfernt. Durch den Stickstoffstrom wurde jedoch die Sublimation des Ammoniumsalzes begünstigt, das wiederum durch ebenfalls mitgeschleppten ETFAA vom Kühler abgewaschen wurde.

Die Lösung wurde nach Beendigung des Zutropfens noch eine Stunde auf 100 °C gehalten um das Salz vollständig zu thermolysieren. Die NMR-Probe nach Reaktionsende zeigt 95 % der Sollkomponente, sowie noch Eduktreste an. In der Kühlfalle wurden neben Wasser wiederum auch noch Spuren von TFK-Hydrat und Ethanol gefunden.

### NMR-Daten:

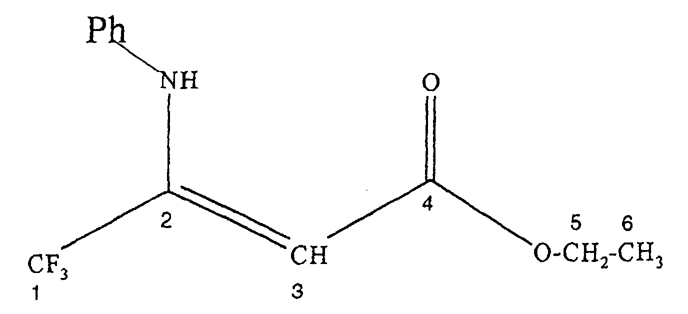

Die NMR Daten von der Substanz zeigen folgende chemische Verschiebungen:
¹³C- NMR ( 100 MHz, CDCl₃): δ (ppm) =120,3 (C-1); 147,1 (C-2); 88,7 (C-3); 169,6 (C-4); 60,1 (C-5); 14,1 (C-6); 125,9; 126,5; 128,8; 138,4 (C-Phenyl).
Ebenso, wie die ¹³C Daten, waren die 400 MHz ¹H im Einklang mit der Sollstruktur.

### Beispiel 12: (Vergleichsbeispiel)

### Thermolyse ohne Inertgas, Löse- oder Schleppmittel

### Thermolyse des NH₄⁺[CF₃COCHCO₂Et]⁻: zum 3-Amino-4,4,4-Trifluorethylcrotonat ohne Inertgas:

In einem 100 ml Dreihalskolben wurden 50 g Ammoniumsalz eingefüllt. Der Kolben wurde mit einem Rückflußkühler (Kühlmedium 70 °C) versehen und in ein auf 120 °C temperiertes Ölbad gegeben. Nach 90 min. war kein Ammoniumsalz mehr nachweisbar. An der Oberfläche der Suspension sind "Fettaugen", herrührend von Wassertropfen, erkennbar. Das NMR-Spektrum zeigt neben dem 3-Amino-4,4,4-Trifluorethylcrotonat das Vorliegen von Nebenkomponenten an. Auch nach Destillation konnte aufgrund von Verunreinigungen nur Sollprodukt mit 97%iger Reinheit und 82%iger Ausbeute isoliert werden.

## Patentansprüche

1. Verfahren zur Herstellung von am C-4-Atom durch Halogen substituierten Crotonaten ausgewählt aus der Gruppe umfassend Niedrigalkyl-3-amino-4,4,4-trifluorocrotonate, Niedrigalkyl-3-amino-4, 4-difluor-4-chlor-crotonate und Niedrigalkyl-3-amino-4,4-difluorcrotonate, wobei die Aminogruppe gegebenenfalls durch 1 oder 2 C1-C3-Alkylgruppen oder Arylgruppen substituiert sein kann und Niedrigalkyl Methyl, Ethyl, n-Propyl und i-Propyl bedeutet, wobei man unter thermolytischen Bedingungen ohne Zusatz einer Säure die simultane Bildung von Wasser und am C-4-Atom durch Halogen substituierten Niedrigalkyl-3-amino-crotonaten bewirkt, und wobei man gebildetes Wasser aus der Reaktionsmischung abtrennt, indem man vom entsprechenden Ammoniumsalz des am C-4-Atom durch Halogen substituierten Acetessigsäure-Niedrigalkylesters ausgeht und in Anwesenheit eines Schleppmittels für das gebildete Wasser thermolysiert oder indem man in Abwesenheit eines Lösungsmittels Inertgas durch das geschmolzen vorliegende Ammoniumsalz des am C-4-Atom durch Halogen substituierten Acetessigsäure-Niedrigalkylesters leitet und dadurch gebildetes Wasser aus der Reaktionsmischung abführt.

2. Verfahren nach Anspruch 1, **dadurch gekennzeichnet, daß** man an der Aminogruppe durch Wasserstoff substituiertes Niedrigalkylcrotonat, insbesondere Niedrigalkyl-3-amino-4,4,4-trifluorocrotonat herstellt.

3. Verfahren nach Anspruch, 1 oder 2, **dadurch gekennzeichnet, daß** Niedrigalkyl für Methyl oder Ethyl steht.

4. Verfahren nach Anspruch 1, **dadurch gekennzeichnet, daß** man in Anwesenheit eines Schleppmittels thermolysiert.

5. Verfahren nach Anspruch 4, **dadurch gekennzeichnet, daß** man als Schleppmittel aromatische Kohlenwasserstoffe oder halogenierte Kohlenwasserstoffe einsetzt.

6. Verfahren nach Anspruch 4, **dadurch gekennzeichnet, daß** man ein Schleppmittel verwendet, welches spezifisch schwerer als die sich ausbildende Wasserphase ist.

7. Verfahren nach Anspruch 6, **dadurch gekennzeichnet, daß** man Trichlorethylen oder Tetrachlorkohlenstoff als Schleppmittel einsetzt.

8. Verfahren nach Anspruch 4, **dadurch gekennzeichnet, daß** man das Ammoniumsalz des Crotonats in-situ herstellt und seine Herstellung und die Thermolyse simultan durchführt.

9. Verfahren nach Anspruch 4, **dadurch gekennzeichnet, daß** man es kontinuierlich durchführt.

10. Verfahren nach Anspruch 1, **dadurch gekennzeichnet, daß** man Inertgas durch das geschmolzene Salz leitet.

11. Verfahren nach Anspruch 10, **dadurch gekennzeichnet, daß** man bei einer Temperatur im Bereich vom Schmelzpunkt des Salzes bzw. der Reaktionsmischung bis hin zu einer Temperatur von maximal 105 °C arbeitet.

12. Verfahren nach Anspruch 11, **dadurch gekennzeichnet, daß** man im Bereich von 80 bis 95 °C arbeitet.

13. Verfahren nach Anspruch 10, **dadurch gekennzeichnet, daß** man als Inertgas Stickstoff, Argon, Helium, CO₂ oder deren Gemische einsetzt.

14. Verfahren nach Anspruch 10, **dadurch gekennzeichnet, daß** man das Ammoniumsalz in einer ersten Stufe durch Einleiten von Ammoniak in und/oder über den am C-4-Atom durch Halogen substituierten Acetessigsäure-Niedrigalkylester herstellt und die Thermolyse in einer zweiten Stufe durchführt.

15. Verfahren nach Anspruch 10, **dadurch gekennzeichnet, daß** man die in-situ-Herstellung des Ammoniumsalzes des am C-4-Atom durch Halogen substituierten. Acetessigsäure-Niedrigalkylesters und seine Thermolyse unter Bildung von Wasser und von am C-4-Atom durch Halogen substituiertem Niedrigalkyl-3-amino-crotonat simultan durchführt.

16. Verfahren nach Anspruch 10, **dadurch gekennzeichnet, daß** man es kontinuierlich durchführt.

17. Verfahren nach Anspruch 1, **dadurch gekennzeichnet, daß** man das Ammoniumsalz des 4,4,4-Trifluoracetessigsäure-Niedrigalkylesters zu Niedrigalkyl-3-amino-4,4,4-trifluorcrotonat umsetzt.

## Claims

1. A process for the preparation of crotonates substituted at the C4 atom by halogen, selected from the group comprising lower-alkyl-3-amino-4,4,4-trifluorocrotonates, lower-alkyl-3-amino-4,4-difluoro-4-chlorocrotonates and lower-alkyl-3-amino-4,4-difluorocrotonates, wherein the amino group may optionally be substituted by 1 or 2 C1-C3-alkyl groups or aryl groups and lower alkyl stands for methyl, ethyl, n-propyl and i-propyl, wherein, under thermolytic conditions without the addition of an acid the simultaneous formation of water and lower-alkyl-3-amino-crotonates substituted at the C4 atom by halogen is effected, and wherein resulting water is separated off from the reaction mixture, in that the point of departure is the corresponding ammonium salt of the acetoacetic acid lower-alkyl ester which is substituted at the C4 atom by halogen, and thermolysis is effected in the presence of an entraining agent for the resulting water, or in that in the absence of a solvent inert gas is passed through the molten ammonium salt of the acetoacetic acid lower-alkyl ester which is substituted at the C4 atom by halogen, and resulting water is removed from the reaction mixture.

2. A process according to Claim 1, **characterised in that** lower-alkyl crotonate substituted at the amino group by hydrogen, in particular lower-alkyl-3-amino-4,4,4-trifluorocrotonate, is prepared.

3. A process according to Claim 1 or 2, **characterised in that** lower alkyl stands for methyl or ethyl.

4. A process according to Claim 1, **characterised in that** thermolysis is effected in the presence of an entraining agent.

5. A process according to Claim 4, **characterised in that** aromatic hydrocarbons or halogenated hydrocarbons are used as entraining agent.

6. A process according to Claim 4, **characterised in that** an entraining agent is used which has a greater specific gravity than the aqueous phase which forms.

7. A process according to Claim 6, **characterised in that** trichloroethylene or carbon tetrachloride is used as entraining agent.

8. A process according to Claim 4, **characterised in that** the ammonium salt of the crotonate is prepared *in situ* and the preparation thereof and the thermolysis are effected simultaneously.

9. A process according to Claim 4, **characterised in that** it is performed continuously.

10. A process according to Claim 1, **characterised in that** inert gas is passed through the molten salt.

11. A process according to Claim 10, **characterised in that** operation is at a temperature in the range of the melting point of the salt or of the reaction mixture up to a temperature of at most 105°C.

12. A process according to Claim 11, **characterised in that** operation is in the range from 80 to 95°C.

13. A process according to Claim 10, **characterised in that** nitrogen, argon, helium, CO₂ or mixtures thereof are used as inert gas.

14. A process according to Claim 10, **characterised in that** the ammonium salt is prepared in a first stage by introducing ammonia into and/or over the acetoacetic acid lower-alkyl ester substituted at the C4 atom by halogen and the thermolysis is performed in a second stage.

15. A process according to Claim 10, **characterised in that** the *in situ* preparation of the ammonium salt of the acetoacetic acid lower-alkyl ester substituted at the C4 atom by halogen and its thermolysis are performed simultaneously, forming water and lower-alkyl-3-amino-crotonate substituted at the C4 atom by halogen.

16. A process according to Claim 10, **characterised in that** it is performed continuously.

17. A process according to Claim 1, **characterised in that** the ammonium salt of the 4,4,4-trifluoroacetoacetic acid lower-alkyl ester is reacted to form lower-alkyl-3-amino-4,4,4-trifluorocrotonate.

## Revendications

1. Procédé de préparation de crotonates substitués par un halogène sur l'atome C₄, choisis dans l'ensemble comprenant les 3-amino-4,4,4-trifluorocrotonates d'alkyle inférieur, les 3-amino-4,4-difluoro-4-chlorocrotonates d'alkyle inférieur et les 3-amino-4,4-difluorocrotonates d'alkyle inférieur, le groupe amino pouvant éventuellement être substitué par un ou deux groupes alkyle en C₁-C₃ ou aryle, "alkyle inférieur" désignant les groupes méthyle, éthyle, n-propyle et isopropyle, procédé dans lequel, dans des conditions thermolytiques et sans addition d'un acide, on provoque la formation simultanée d'eau et de 3-amino-crotonates d'alkyle inférieur substitués par un halogène sur l'atome C₄, et on sépare du mélange réactionnel l'eau qui se forme, où on part du sel d'ammonium correspondant de l'ester alkylique inférieur de l'acide acétoacétique substitué par un halogène sur l'atome C₄, et on procède à une thermolyse en présence d'un agent d'entraînement pour l'eau formée, ou encore, en l'absence d'un solvant, on fait passer un gaz inerte à travers le sel d'ammonium, présent à l'état fondu, de l'ester alkylique inférieur de l'acide acétoacétique substitué par un halogène sur l'atome C₄, l'eau formée à cette occasion étant évacuée du mélange réactionnel.

2. Procédé selon la revendication 1, **caractérisé en ce qu'**on prépare un crotonate d'alkyle inférieur substitué par un atome d'hydrogène sur le groupe amino, en particulier un 3-amino-4,4,4-trifluorocrotonate d'alkyle inférieur.

3. Procédé selon la revendication 1 ou 2, **caractérisé en ce que** le groupe alkyle inférieur est le groupe méthyle ou éthyle.

4. Procédé selon la revendication 1, **caractérisé en ce qu'**on procède à la thermolyse en présence d'un agent d'entraînement.

5. Procédé selon la revendication 4, **caractérisé en ce qu'**on utilise en tant qu'agent d'entraînement des hydrocarbures aromatiques ou des hydrocarbures halogénés.

6. Procédé selon la revendication 4, **caractérisé en ce qu'**on utilise un agent d'entraînement qui a une densité supérieure à celle de la phase aqueuse qui se forme.

7. Procédé selon la revendication 6, **caractérisé en ce qu'**on utilise en tant qu'agent d'entraînement le trichloréthylène ou le tétrachlorure de carbone.

8. Procédé selon la revendication 4, **caractérisé en ce qu'**on prépare in situ le sel d'ammonium du crotonate, et on met en oeuvre simultanément sa préparation et la thermolyse.

9. Procédé selon la revendication 4, **caractérisé en ce qu'**on le met en oeuvre d'une manière continue.

10. Procédé selon la revendication 1, **caractérisé en ce qu'**on fait passer un gaz inerte à travers le sel fondu.

11. Procédé selon la revendication 10, **caractérisé en ce qu'**on travaille à une température comprise entre le point de fusion du sel ou du mélange réactionnel et une température maximale de 105°C.

12. Procédé selon la revendication 11, **caractérisé en ce qu'**on travaille dans la plage de 80 à 95°C.

13. Procédé selon la revendication 10, **caractérisé en ce qu'**on utilise en tant que gaz inerte l'azote, l'argon, l'hélium, le CO₂ ou leurs mélanges.

14. Procédé selon la revendication 10, **caractérisé en ce qu'**on prépare le sel d'ammonium dans une première étape, en envoyant de l'ammoniac dans et/ou sur l'ester alkylique inférieur de l'acide acétoacétique substitué par un atome d'halogène sur l'atome C₄ et **en ce que** dans une deuxième étape, on met en oeuvre la thermolyse.

15. Procédé selon la revendication 10, **caractérisé en ce qu'**on met en oeuvre simultanément la préparation in situ du sel d'ammonium de l'ester alkylique inférieur de l'acide acétoacétique substitué par un halogène sur l'atome C₄ et sa thermolyse avec formation d'eau et d'un 3-aminocrotonate d'alkyle inférieur substitué par un halogène sur l'atome C₄.

16. Procédé selon la revendication 10, **caractérisé en ce qu'**il est mis en oeuvre en continu.

17. Procédé selon la revendication 1, **caractérisé en ce qu'**on fait réagir le sel d'ammonium de l'ester alkylique inférieur de l'acide 4,4,4-trifluoroacétoacétique pour obtenir un 3-amino-4,4,4-trifluorocrotonate d'alkyle inférieur.
